# EUROPEAN PATENT SPECIFICATION

(11) **EP 3 528 625 B1**
(45) Date of publication and mention of the grant of the patent: **27.01.2021**
(21) Application number: 17861538.1
(22) Date of filing: 17.10.2017
(51) Int. Cl.: A01N 1/00, A01N 1/02, B01L 3/00, B01L 9/00, B81B 3/00, C12M 1/00, C12M 1/34

(54) **MICROFLUIDICS AEROSOL-EVALUATION APPARATUS**
MIKROFLUIDISCHE AEROSOLAUSWERTUNGSVORRICHTUNG
APPAREIL D'ÉVALUATION D'AÉROSOL MICROFLUIDIQUE

(30) Priority: 19.10.2016 US 201615297836
(43) Date of publication of application: 28.08.2019
(73) Proprietor: R. J. Reynolds Tobacco Company, Winston-Salem, NC 27101-3804 (US)
(72) Inventor: SEARS, Stephen B., Siler City North Carolina 27344 (US); SPOO, Wayne, Pfafftown North Carolina 27040 (US); FIELDS, Wanda, Kernersville 27284 (US)
(74) Representative: Hoeger, Stellrecht & Partner Patentanwälte mbB
(86) International application number: PCT/US2017/057016
(87) International publication number: WO 2018/075543

(56) References cited:
- WO-A1-2013/085909
- WO-A2-2015/138034
- US-A- 4 620 965
- US-A1- 2009 075 282
- US-A1- 2012 182 609
- US-A1- 2013 004 386
- US-A1- 2015 004 077
- NAHAR KAMRUN ET AL: "In vitro,in vivoandex vivomodels for studying particle deposition and drug absorption of inhaled pharmaceuticals", EUROPEAN JOURNAL OF PHARMACEUTICAL SCIENCES, ELSEVIER, AMSTERDAM, NL, vol. 49, no. 5, 22 June 2013 (2013-06-22), pages 805-818, XP028683989, ISSN: 0928-0987, DOI: 10.1016/J.EJPS.2013.06.004
- BOVARD ET AL.: 'Organs-on-a-Chip: A New Paradigm for Toxicological Assessment and Preclinical Drug Development' TOXICOLOGY RESEARCH AND APPLICATION vol. 1, 17 August 2017, pages 1 - 16, XP055477414
- BENAM ET AL.: 'Small Airway-on-a-Chip Enables Analysis of Human Lung Inflammation and Drug Responses In Vitro' NATURE METHODS vol. 13, no. 2, 01 February 2016, pages 151 - 157, XP055477416
- FIDKOWSKI ET AL.: 'Endothelialized Microvasculature Based on a Biodegradable Elastomer' TISSUE ENGINEERING vol. 11, 01 January 2005, pages 302 - 309, XP009074028
- HUH ET AL.: 'Reconstituting Organ-Level Lung Functions on a Chip' SCIENCE vol. 328, 25 June 2010, pages 1662 - 1668, XP002678090
- MURPHY ET AL.: '3D Bioprinting of Tissues and Organs' NATURE BIOTECHNOLOGY vol. 32, 01 August 2014, pages 773 - 785, XP055244641
- YE ET AL.: 'A Biodegradable Microvessel Scaffold as a Framework to Enable Vascular Support of Engineered Tissues' BIOMATERIALS vol. 34, no. 38, 31 December 2013, pages 10007 - 10015, XP028735924

## Description

### CROSS-REFERENCE TO RELATED APPLICATION

The present application claims priority to U.S. Patent Application No. 15/297,836 filed October 19, 2016.

### FIELD OF THE DISCLOSURE

The present application relates generally to a synthetic organ comprising an organ-on-chip microfluidic device, and related methods of use.

### BACKGROUND

WO 2013/085909 A1 discloses an airway tissue model system, comprising a first chamber having an inlet and an outlet and containing epithelial cells, a second chamber having an inlet and an outlet containing an extracellular matrix, wherein the second chamber is separated from the first chamber by a porous membrane, and a third chamber having an inlet and an outlet, wherein the third chamber is separated from the second chamber by a porous membrane, and wherein the airway tissue model is configured to provide a separate fluidic pathway through each of said first, second and third chambers.

The Article NAHAR KAMRUN ET AL: "In vitro, in vivo and ex vivo models for studying particle deposition and drug absorption on inhaled pharmaceuticals", EUROPEAN JOURNAL OF PHARMACEUTICAL SCIENCES, ELSEVIER, AMSTERDAM, NL, vol. 49, no. 5, 22 June 2013 (2013-06-22). Pages 805-818, XP028683989, ISSN: 0928-0987, DOI: 10.1016/J.EJPS.2013.06.004 describes in vitro, in vivo an ex vivo models to study the behaviors of airborne particles in the lungs and to determine the absorption of drugs after pulmonary administration.

Testing of tobacco aerosol and other aerosolized nicotine products is conducted regularly in the industry. Whole-animal *in vivo* testing is often employed in assessing the properties of tobacco and nicotine products. Lippiello, P. M., et al. "RJR-2403: A nicotinic agonist with CNS selectivity II. In vivo characterization." Journal of Pharmacology and Experimental Therapeutics 279.3 (1996): 1422-1429; Rada, Pedro, Kristin Jensen, and Bartley G. Hoebel. "Effects of nicotine and mecamylamine-induced withdrawal on extracellular dopamine and acetylcholine in the rat nucleus accumbens." Psychopharmacology 157.1 (2001): 105-110; Abdulla, F. A., et al. "Relationship between up-regulation of nicotine binding sites in rat brain and delayed cognitive enhancement observed after chronic or acute nicotinic receptor stimulation." Psychopharmacology 124.4 (1996): 323-331; Bencherif, M., et al. "RJR-2403: a nicotinic agonist with CNS selectivity I. In vitro characterization." Journal of Pharmacology and Experimental Therapeutics 279.3 (1996): 1413-1421; Levin, Edward D., and N. Channelle Christopher. "Persistence of nicotinic agonist RJR 2403-induced working memory improvement in rats." Drug development research 55.2 (2002): 97-103.

The guiding principles underpinning the humane use of animals in scientific research are called the three "R's," first described by W. M. S. Russell and R. L. Burch in 1959. Briefly, researchers should be encouraged to replace the use of animals with alternative techniques, or avoid the use of animals altogether, reduce the number of animals used to a minimum, to obtain information from fewer animals or more information from the same number of animals and refine the way experiments are carried out, to make sure animals suffer as little as possible. Institutional Animal Care and Use Committees (IACUCs) at research facilities should review practices and study plans/protocols with similar goals in mind.

Many companies are often required to conduct *in vitro* ("in glass") and *in vivo* ("in animal") testing in order to comply with company guidelines and federal regulations required for approval of products (pharmaceuticals, medical devices, cosmetics, tobacco products, etc.) prior to market release to prove safety and efficacy. Regulators are aware of, and will accept, relevant data sets using *in vitro* technology, however, in many cases, there are no acceptable substitutes for *in vivo* testing for some data requirements.

*In vitro* testing, such as cell culture techniques, are used extensively in the scientific world today, but have limitations based on the test(s) being conducted, usually related to the fact the cells or tissues being used are isolated and not allowed to interact with other living tissues as they exist in the animal model (lack of multiple organ and systemic interactions).

Given animal testing bans in many countries and the high cost and time requirements of *in vivo* testing, it would be desirable to develop improved methods that are better predictors of toxicity but further limit or do not require the use of animals.

Microfluidics is a field of applied science and engineering that deals with the controlled movement of small volumes of liquid in small spaces. In this context, the phrase "small volumes" typically refers to liquid volumes in the micro-liter, nano-liter, pico-liter or smaller range. Similarly, "small spaces" may refer to geometries on the millimeter (or smaller) scale. To date, microfluidics has found application in important areas of modern technology including chemical sensors, inkjet printing, gene sequencing, fuel-cell design, tissue engineering and lab-on-a-chip diagnostics (including clinical pathology). Devices based upon, or employing, microfluidic principles typically offer the benefits of portability, low energy consumption, high throughput, minimal sample/reagent volumes and ease of automation. Dittrich, Petra S., and Andreas Manz. "Lab-on-a-chip: microfluidics in drug discovery." Nature Reviews Drug Discovery 5.3 (2006): 210-218; Teh, Shia-Yen, et al. "Droplet microfluidics." Lab on a Chip 8.2 (2008): 198-220; Neuži, Pavel, et al. "Revisiting lab-on-a-chip technology for drug discovery" Nature reviews Drug discovery 11.8 (2012): 620-632; Ghaemmaghami, Amir M., et al. "Biomimetic tissues on a chip for drug discovery." Drug discovery today 17.3 (2012): 173-181; Huh, Dongeun, et al. "Microengineered physiological biomimicry: organs-on-chips." Lab on a chip 12.12 (2012): 2156-2164.

Accordingly, it would be desirable to provide a synthetic organ to accommodate cigarette aerosol exposure models, provide the potential to reduce/eliminate the number of animals required to comply with regulations required for approval of products prior to market release to prove safety and efficacy, reduce the time and cost associated with conducting *in vivo* studies, and which is designed for mass throughput allowing testing to be accomplished more quickly, at less expense, and with an accelerated "time-to-fail."

### SUMMARY

In a first aspect, the present disclosure provides a synthetic organ comprising: one or more airways and a first opening configured to introduce aerosol or vapor into the one or more airways; an organ-on-chip microfluidic device having lung/respiratory-tract tissue or cells; and one or more mounting position(s) within the one or more airways. In some embodiments, the mounting position(s) within the one or more airways are configured to accept the organ-on-chip microfluidic device at a position relative to the first opening to replicate specific portions of a lung. In some embodiments, the one or more airways comprises a tube and/or comprises one or more bifurcation(s) and/or replicates at least a portion of a lung passageway, which can be representative of that of a human or mammalian animal model. In some embodiments, the synthetic organ comprises a second opening in communication with an elastic vessel, whereby an increase in pressure within the synthetic organ causes the elastic vessel to expand. In other embodiments, the synthetic organ comprises a plurality of organ-on-chip microfluidic device(s), wherein each organ-on-chip microfluidic device of the plurality comprises a layer (or layers) of lung tissue or cells native to the specific portion of a lung at which it is located. In some embodiments, the organ-on-chip microfluidic device is a passive device or an active device. In other embodiments, the one or more airways comprises a cast of a lung or a 3-D printing of a portion of a respiratory tract of a lung. In yet another embodiment, the organ-on-chip microfluidic device comprises one or more than one layer of differentiated lung tissue. In some embodiments, the one or more airways is constructed with an elastic material. In some embodiments, the synthetic organ further comprises a control device configured to control variables, such as temperature, humidity and/or concentration of aerosol, within the device and/or further comprises a programmable aerosol or vapor producing machine in communication with the first opening for the purpose of simulating a human puff profile and/or further comprises a filter covering an opening on the organ-on-chip microfluidic device whereby the filter allows a gas phase of a vapor, aerosol, or other airborne material (e.g., aerosol particles) to enter the chip.

In a second aspect, a method of evaluating a vapor, aerosol, or other airborne material product (e.g., a tobacco product) is provided, wherein the method comprises: providing a synthetic organ having an organ-on-chip microfluidic device comprising lung tissue or cells; and introducing a vapor, aerosol, or other airborne material product (e.g., tobacco aerosol) through a first opening of the synthetic organ. In some embodiments, the organ is a synthetic organ of one or more of the embodiments disclosed herein. In some embodiments, the method further comprises evaluating a response of the one or more organ-on-chip microfluidic devices to the vapor, aerosol, or other airborne material.

In a third aspect, a method for mimicking an *in vivo* testing systems for a vapor, aerosol, or other airborne material product (e.g., a tobacco product) is provided, wherein the method comprises: providing a synthetic organ having an organ-on-chip microfluidic device comprising lung tissue or cells; and introducing vapor, aerosol, or other airborne material product (e.g., tobacco aerosol) into the synthetic organ. In some embodiments, the organ is a synthetic organ of one or more of the embodiments disclosed herein. In some embodiments, the method further comprises evaluating a response of the one or more organ-on-chip microfluidic devices to the vapor, aerosol, or other airborne material product.

Further features and advantages of the present disclosure are set forth in more detail in the following description.

### BRIEF DESCRIPTION OF THE DRAWINGS

Having thus described the disclosure in general terms, reference will now be made to the accompanying drawings, which are not necessarily drawn to scale, and wherein:

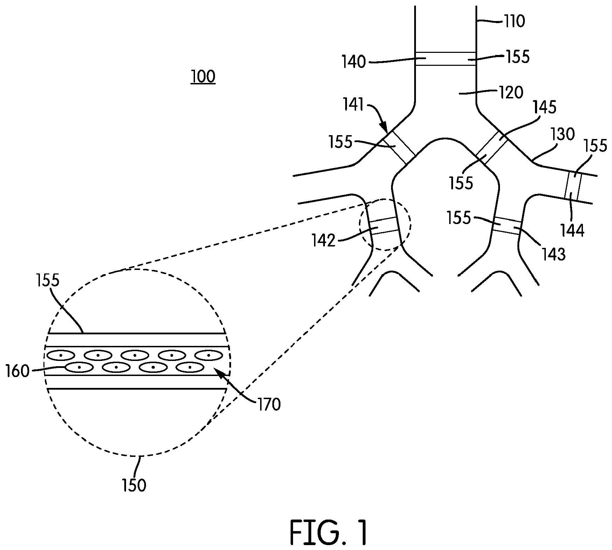

FIG. 1 is a view of a synthetic organ, according to an illustrative embodiment of the present disclosure.

### DETAILED DESCRIPTION OF THE DISCLOSURE

Various embodiments are described hereinafter. It should be noted that the specific embodiments are not intended as an exhaustive description or as a limitation to the broader aspects discussed herein. One aspect described in conjunction with a particular embodiment is not necessarily limited to that embodiment and can be practiced with any other embodiment(s).

As used herein, "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 15% of the particular term.

The use of the terms "a" and "an" and "the" and similar referents in the context of describing the elements (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*e.g.*, "such as") provided herein, is intended merely to better illuminate the embodiments and does not pose a limitation on the scope of the claims unless otherwise stated. No language in the specification should be construed as indicating any non-claimed element as essential.

Various embodiments described herein relate to a synthetic organ (e.g., a synthetic lung) that includes one or more airways and a first opening in communication with the one or more airways, optionally an organ-on-chip microfluidic device having tissue or cells (e.g., lung tissue or cells), and one or more mounting position(s) within the airway configured to accept the organ-on-chip microfluidic device. The mounting position(s) within the airway are configured to accept the organ-on-chip microfluidic device at a position(s) relative to the first opening to replicate specific portions of the synthetic organ.

In exemplary embodiments, the synthetic organ includes one or more airways in communication with the first opening that is configured to introduce a vapor, aerosol, or other airborne material into the one or more airways. The one or more airways will be employed to transport the vapor, aerosol, or other airborne material to the microfluidic chip entry ports contained on organ-on-chip microfluidic devices disposed therein. The first opening may also be associated with an entry chamber that simulates the mouth in which the aerosol or vapor may be aged before entry into the airway(s). Accordingly, in some embodiments, the synthetic organ further includes an entry chamber.

In some embodiments, the one or more airways have a generally cylindrical shape with the first opening on or near one end of the cylinder and the organ-on-chip microfluidic device at a predetermined distance from the first opening.

After introducing an amount of vapor, aerosol, or other airborne material to the synthetic organ, entrance to the one or more airways may be controlled by use of an entry chamber to allow aging of the vapor, aerosol, or other airborne material for a predetermined amount of time. The entry chamber design and control may also allow/determine a desired dilution of the vapor, aerosol or other airborne material as well as a predetermined distance before reaching one or more organ-on-chip microfluidic device in the airway. It is to be understood that the predetermined distance and flow rate may be adjusted according to the user's desired aging time for the introduced vapor, aerosol, or other airborne material. It may also be adjusted to conform to a desired "puffing regimen" or smoking protocol prescribed for a vapor/aerosol producing product (e.g., cigarette, electronic cigarette, cigar, etc.) Further, other factors may be modified to mimic certain conditions, such as those in the respiratory tract of a mammal's lung, including the adjustment of temperature or relative humidity. For example, the temperature and/or relative humidity are adjusted to mimic desired variables in the human or animal respiratory tract of a lung.

The predetermined distance described above may vary depending on various parameters the user wishes to study. For example, the predetermined distance from the first opening to a mounting position configured to accept the organ-on-chip microfluidic device may be set to explore or mimic the effect of a vapor, aerosol, or other airborne material on certain portions of a respiratory tract or lung -- for example, at least a portion of the respiratory tract of the lung or a particular level of a bronchial tree within a lung. Accordingly, in some embodiments, the identity of the lung tissue used in organ-on-chip device may be set to include lung tissue from a particular level of a bronchial tree within a lung. Lung tissue varies by the depth of the tissue from the entry point into the air passageways in a body. So too, the synthetic organ described above may have one or more types or layers of lung tissue in the synthetic organ corresponding to the depth for which the lung tissue is representative. Accordingly, the predetermined distance from the first opening configured to introduce a vapor, aerosol, or other airborne material to the mounting positions within the one or more airways configured to accept the organ-on-chip microfluidic device may be determined by the distance of the natural tissue in the lung. These distances can vary greatly between species (i.e., human vs. rat) due to age, genetics, diets, etc., as well as between animals within the same genus and species. By example, Table 1 is adapted from Mrudula (2011) and demonstrates this variability within humans, with similar variability expected in laboratory animals. This same variability can be expected in airway below the main bronchial tree.

The vapor, aerosol, or other airborne material may be any material that is intended to be exposed to a respiratory tract of a lung, the effects of which on lung tissue *in vivo*, may be modeled with the synthetic organ *in vitro.* For example, to model the effects of smoking on lung tissue, the vapor, aerosol, or other airborne material may be a tobacco-based vapor or aerosol, such as aerosol from an electronic cigarette. The tobacco or electronic cigarette-based vapor or aerosol may be from cigarette, pipe, cigar, or other tobacco-based products as well. Additional non-limiting examples of vapors, aerosols, or other airborne materials that may be employed in exemplary embodiments of the disclosure include environmental toxins or pollutant, airborne chemicals, drugs or drug candidates, airborne particles including pollen, viruses, bacteria and unicellular organisms.

**Table 1. Trachea and bronchial tree measurement in humans**

| | **Trachea** | | **Main Bronchus** | |
|---|---|---|---|---|
| | Length (cm) | Width (cm) | Right (cm) | Left (cm) |
| Range | 6.1-9.6 | 1.2-2.5 | 1-2.4 | 2.9-4.2 |
| Average | 7.87 | 1.99 | 2.28 | 3.86 |

Mainstream cigarette aerosol (MSS) exposure models may be used with the system. Smoking machines, for example, Borgwaldt RM20 or linear smoking machines, may be used. Aerosol generators designed or adapted to produce aerosol from electronic cigarettes can also be used. Procedures and smoking devices developed for the analysis of cigarette aerosol or other aerosol/ aerosol product may be employed in the present embodiments. See, *e.g.*, ISO 3308: Routine analytical cigarette smoking machine - Definitions and standard conditions; Reference number ISO 3308:1991 (E), International Organization for Standardization, Geneva, Switzerland, 1991; ISO 3402: Tobacco and Tobacco Products - Atmosphere for conditioning and testing; Reference number ISO 3402:1991(E), International Organization for Standardization, Geneva, Switzerland, 1991; ISO 4387: Cigarettes - Determination of total and nicotine free dry particulate matter using a routine analytical smoking machine; Reference number ISO 4387:1991 (E), International Organization for Standardization, Geneva, Switzerland, 1991. See also CORESTA Recommended Method N° 81. Routine Analytical Machine For E-Cigarette Aerosol Generation And Collection - Definitions And Standard Conditions (June 2015). Negative-pressure and positive-pressure machines may serve to expose the synthetic respiratory tract or lung to the vapor, aerosol or other airborne material. The cigarette or tobacco device to be tested may be selected by the skilled artisan, and may include for example, reference cigarettes known in the art. See Davies, H.M. and A. Vaught: The reference cigarette: Kentucky Tobacco Research & Development Center (KTRDC), Lexington, KY, 2003, or see https://www.coresta.org/sites/default/files/technical_documents/main/CRM_81.pdf.

The one or more airways of the synthetic organ may be designed to mimic at least a portion of the respiratory tract of a lung. It is understood that in the respiratory tract of a lung, the trachea bifurcates into the primary bronchi, which branch into smaller, secondary and tertiary bronchi that ultimately branch into still smaller bronchioles. Accordingly, in some embodiments, the one or more airways is a generally cylindrical or tubular shape and comprises at least one bifurcation. In some embodiments, the one or more airways comprises at least 1, 2, 3, 4, 5, 6 or 7 bifurcations (e.g., the one or more airways comprises a network of larger and smaller airways). In some embodiments, the diameter of the generally cylindrical or tubular one or more airways is reduced with each successive bifurcation, thereby more closely mimicking the respiratory tract structure of a lung. In some embodiments, the one or more airways comprises one airway. In other embodiments, the one or more airways comprises 2, 3, 4, 5, 6, 7, 8, 9, 10, 11, 12, 13, 14 or 15 or more airways. In some embodiments, the multiple airways are a network of macro- and/or microfluidic airways.

To replicate the respiratory tract of a lung structure more closely, the one or more airways of the synthetic organ may be produced from a physical cast of the respiratory tract of at least a portion of a lung, for example a human, dog, or rat lung using well-described techniques. In other embodiments, the one or more airways of the synthetic organ may be produced via 3-D printing. Where 3-D printing is employed, the print may be based upon an image of a lung, for example a Magnetic Resonance Image (MRI), or other similar technology, of a lung. Casts of a selected lung may be made by methods known in the art, for example, see Frank, N. R., and R. E. Yoder "A method of making a flexible cast of the lung" Journal of Applied Physiology 21.6 (1966): 1925-1926; Yeh, Hsu-Chi, and G. M. Schum "Models of human lung airways and their application to inhaled particle deposition" Bulletin of Mathematical Biology 42.3 (1980): 461-480; Yu, C. P., and C. K. Diu "A comparative study of aerosol deposition in different lung models" The American Industrial Hygiene Association Journal 43.1 (1982): 54-65; Zhou, Yue, and Yung-Sung Cheng "Particle deposition in a cast of human tracheobronchial airways" Aerosol Science and Technology 39.6 (2005): 492-500; Carson, James P., et al. "High resolution lung airway cast segmentation with proper topology suitable for computational fluid dynamic simulations" Computerized Medical Imaging and Graphics 34.7 (2010): 572-578; Brücker, Ch, and W. Schroder "Flow visualization in a model of the bronchial tree in the human lung airways via 3-D PIV" Proceedings of PSFVIP-4, Chamonix, France (2003): 3-5; Tsuda, Akira, et al. "Finite element 3D reconstruction of the pulmonary acinus imaged by synchrotron X-ray tomography" Journal of Applied Physiology 105.3 (2008): 964-976; Tam, Matthew David, et al. "3-D printouts of the tracheobronchial tree generated from CT images as an aid to management in a case of tracheobronchial chondromalacia caused by relapsing polychondritis" Journal of radiology case reports 7.8 (2013): 34.

The above structure of the synthetic organ, *e.g.* lung, may be better understood by referring to FIG. 1. FIG. 1 is a view of an illustrative embodiment where the one or more airways is designed to mimic a portion of a lung, including the trachea and several bifurcations. As illustrated in FIG. 1, the lung 100 starts with a trachea type structure 110 that bifurcates into to the bronchial stages 120, which then further bifurcates 130 in other branching structures of further bronchial stages and eventually bronchioles. At various points throughout the structure 140, 141, 142, 143, 144, 145, there are mounting positions within the one or more airways configured to accept the organ-on-chip microfluidic device. Generic representations of organ-on-chip microfluidic devices 155 are shown at each of mounting positions 140, 141, 142, 143, 144, and 145. FIG. 1 shows a blow up of cells within the organ-on-chip microfluidic device 155 containing lung cells normally found in the respective portion of the lung. In the blowup section, modeling of an organ-on-chip microfluidic device 155 mounted to the bronchial wall 150, cells generally 160, and epithelial cells 170, are shown as contained within the organ-on-chip microfluidic device. In some embodiments, the microfluidic device 155 is mounted as a band of chips around the circumference of the bronchial wall 150. In other embodiments, the microfluidic device 155 is mounted so as to run a portion of the length of the bronchial wall 150. Yet other embodiments include where the microfluidic device 155 is mounted so as to span the opening of the bronchial wall 150.

The one or more airways may be further configured to mimic the expandability, humidity, or other feature of a lung. In some embodiments, the flow of the environment within the one or more airways may be controlled. For example, a portion of the one or more airways may be configured to expand and contract. This expansion and contraction may be, for example, capable of mimicking the expansion and contraction of an airway within a lung.

The first opening configured to introduce a vapor, aerosol, or other airborne material, for example, aerosol into the one or more airways may be in communication with a source of aerosol, for example, a puffing machine or other device configured to produce an aerosolized plume of tobacco aerosol or other aerosolized nicotine product. Referring to FIG. 1, an illustrative embodiment is shown where the first opening is the trachea leading into or connecting to one or more airways. The aerosol, such as aerosol or other vapor or aerosolized nicotine product is introduced through the trachea of the one or more airways. Alternatively, the first opening/entry-chamber may mimic the human oral cavity.

The synthetic organ may have one or more second openings designed to mimic realistic flow within the one or more airways. This flow may be adjusted to simulate environmental conditions (temperature, humidity, pressure, etc.) within a breathing lung. The second opening may be configured in communication with an expandable vessel, such as an elastic vessel, whereby an increase in pressure within the synthetic organ causes the elastic vessel to expand. The positioning of the second opening is not limited. In some embodiments, the second opening is at the opposite end of the one or more airways from the first opening. Referring to FIG. 1, a view of an illustrative embodiment where the first one or more second openings of the airway can be at the end of each of the bifurcated airway portions.

### Organ-on-chip microfluidic device

The synthetic organ optionally includes an organ-on-chip microfluidic device having lung tissue or cells. The organ-on-chip technology allows for cell populations to be integrated onto silicon wafers, and oriented to replicate more realistic living and exposure environments found in *in vivo* exposure systems. Organ-on-chip microfluidic devices are known in the art, for example, in PCT Publication Nos. WO/2013/086486, WO/2013/086502, and WO/2010/009307. These devices, however, do not provide for a configuration to allow for realistic simulation of vapor/aerosol dynamical phenomena. For example, the devices do not account for aerosol entering the respiratory tract nor its subsequent aging, dispersion or evolution of the aerosol. There are many ways to "classify" microfluidics devices; but a convenient way in the context of the current disclosure centers on the "forces" used to move the fluid. In particular, "passive" microfluidic devices rely upon surface or capillary forces to move the liquid. These in turn depend upon the properties of the liquids and surfaces employed in the device, as well as liquid/surface interactions (e.g., viscosities, contact angles, surface structure, densities, geometries, etc.). "Active" microfluidic devices rely upon external forces (often in addition to capillary forces) to move the fluid. Examples of external forces are mechanical action (*e.g.*, a micro-electro-mechanical-systems (MEMS) pump, or possibly a "responsive polymeric" material), thermal gradients and electric fields. In addition, micro-valves of various types may also be employed to activate/deactivate and control liquid flow (direction, volume, flow rate) in micro-channels. For example, in certain embodiments, the microfluidic device is an active device. In other embodiments, the microfluidic device is a passive device.

The microfluidic device comprises lung tissue or cells. In some embodiments, the lung tissue or cells comprise a specific type of lung tissue or cells, for example, tissue containing one or more of ciliated epithelial cells, goblet cells, basal cells, squamous epithelium, basal lamina, and epithelium. In some embodiments, the microfluidic device comprises more than one layer of differentiated lung tissue, for example, ciliated epithelial cells, goblet cells, basal cells, squamous epithelium, basal lamina, and epithelium.

The microfluidic device may be designed to be configured in a particular area within the one or more airways. For example, in some embodiments, the microfluidic device is configured on a wall of, or within, the one or more airways. In other embodiments, the microfluidic device is configured to provide a band of cells or tissue running the circumference of the inner diameter of the wall of the one or more airways.

Referring to FIG. 1, a view of an illustrative embodiment where the band of cells or tissue runs the circumference of the inner wall of the airway is provided. Multiple bands of cells may be positioned within different portions of the airway. Each of these bands may have a cell population that reflects the type of cells in the particular portion of the lung. The cells may be mounted on a ribbon-type support (single continuous chip) that circles the entire inner perimeter of the airway or may be mounted in discrete smaller chips at one or more circumferential positions around the airway. The cells may also be nourished by an oxygen/nutrient solution (e.g., "blood proxy") from below.

Transport of the vapor, aerosol, or other airborne material to the lung cells may involve either microfluidic action, by which passive/active forces serve to provide flow of condensed aerosol liquid from the microchip entrance to the cells through capillary passageways, and/or direct deposition of the aerosol/vapor/airborne material on the cell surfaces. The second mechanism may be more representative of molecular vapors and very small aerosol particles.

Optionally, the synthetic organ may be in communication with an "entry" or control chamber, wherein the entry chamber is different from the one or more airways, and may be used, e.g., to age or alter the vapor, aerosol, or other airborne material to simulate a particular environment. For example, an entry chamber may be used to simulate a second-hand aerosol. In some embodiments, the entry chamber may serve as a device to allow the vapor, aerosol, or other airborne material to be present for a predetermined amount of time prior to entering the synthetic organ, a predetermined dilution prior to entering the synthetic organ, and/or a predetermined distance prior to entering the synthetic organ. It is to be understood that the predetermined distance and flow rate may be adjusted according to the user's desired aging time for the introduced vapor, aerosol, or other airborne material. Further, other factors may be modified to mimic certain conditions, such as the adjustment of temperature or relative humidity.

In various embodiments, the invention described herein relates to a method of evaluating a tobacco product with a synthetic organ as described herein.

The method of evaluating a tobacco product may include providing a synthetic organ of the embodiments disclosed herein having an organ-on-chip microfluidic device comprising lung tissue or cells; and introducing a vapor, aerosol, or other airborne material (e.g., tobacco aerosol or aerosolized tobacco product) through a first opening of the synthetic organ. The method may also include evaluating a response of the one or more organ-on-chip microfluidic devices to the vapor, aerosol, or other airborne material aerosol.

It will be appreciated that the amount and manner in which the vapor, aerosol, or other airborne material is introduced can be varied depending on the conditions to be tested. For example, when the vapor or aerosol comprises tobacco aerosol (or some other aerosol) the conditions may be adjusted to simulate the aerosol delivered from a cigarette/smoking device to its user, or aerosol delivered from a cigarette/smoking device to a bystander. Parameters such as vapor, aerosol, or other airborne material concentration (expressed as a mass per unit of volume), flow (volume per unit of time), nicotine concentration and puff-by-puff variations in vapor, aerosol, or other airborne material concentration (among many others) could be used to vary the exposure parameters. In some embodiments, the entry chamber is utilized to set the conditions to be tested.

In various embodiments, the invention described herein relates to a method of mimicking an *in vivo* testing system for tobacco products.

The method of mimicking an *in vivo* testing system for tobacco products may comprise providing a synthetic organ of the embodiments disclosed herein having an organ-on-chip microfluidic device comprising lung tissue or cells; and introducing a vapor, aerosol, or other airborne material (e.g., tobacco aerosol or an aerosolized tobacco product) through a first opening of the synthetic organ. The method may also include evaluating a response of the one or more organ-on-chip microfluidic devices to the vapor, aerosol, or other airborne material (e.g., tobacco aerosol or aerosolized tobacco product).

While certain embodiments have been illustrated and described, it should be understood that changes and modifications can be made therein in accordance with ordinary skill in the art without departing from the technology in its broader aspects as defined in the following claims.

The embodiments, illustratively described herein may suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the claimed technology. Additionally, the phrase "consisting essentially of' will be understood to include those elements specifically recited and those additional elements that do not materially affect the basic and novel characteristics of the claimed technology. The phrase "consisting of' excludes any element not specified.

The present disclosure is not to be limited in terms of the particular embodiments described in this application. Many modifications and variations can be made without departing from its spirit and scope, as will be apparent to those skilled in the art. Functionally equivalent methods and compositions within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is to be understood that this disclosure is not limited to particular methods, compositions or biological systems, which can of course vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a non-limiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member.

Other embodiments are set forth in the following claims.

## Claims

1. A synthetic organ (100) comprising:
one or more airways; and
a first opening in communication with the one or more airways configured to introduce a vapor, aerosol, or other airborne material aerosol into the one or more airways;
an organ-on-chip microfluidic device (155) having lung tissue or cells (160); and
one or more mounting position(s) (140, 141, 142, 143, 144, 145) within the one or more airways configured to accept the organ-on-chip microfluidic device (155).

2. The synthetic organ (100) of claim 1, with at least one of the following:
- the one or more airways comprise a tube;
- the one or more airways comprise one or more bifurcations (130) ;
- the one or more airways replicate at least a portion of a lung passageway, in particular wherein the portion of a lung passageway is representative of a mammal;
- the walls of the one or more airways are elastic, whereby an increase or decrease in pressure within the one or more airways causes the one or more airways to expand or contract;
- the one or more airways comprise a cast of a portion of a respiratory tract of a lung;
- the one or more airways comprise a 3-D printing of a portion of a respiratory tract of a lung;
- the one or more airways is constructed with an elastic material.

3. The synthetic organ (100) of claim 1, comprising a plurality of organ-on-chip microfluidic device(s) (155), distributed throughout the one or more airways, wherein each organ-on-chip microfluidic device (155) of the plurality comprises a layer(s) of lung tissue or cells (160) native to the specific portion of a lung at which it is located.

4. The synthetic organ (100) of claim 3, wherein the organ-on-chip microfluidic device (155) is a passive device or an active device.

5. The synthetic organ (100) of claim 3, wherein the vapor, aerosol, or other airborne material comprises vapor or aerosol comprising nicotine (e.g., tobacco aerosol).

6. The synthetic organ (100) of claim 3, wherein the organ-on-chip microfluidic device (155) comprises one or more layers of differentiated lung tissue.

7. The synthetic organ (100) of claim 1, further comprising a control device configured to control temperature, humidity and/or concentration of the vapor, aerosol, or other airborne material within the device.

8. The synthetic organ (100) of claim 1, further comprising a programmable aerosol or vapor producing machine in communication with the first opening for the purpose of simulating a human puff profile.

9. The synthetic organ (100) of claim 3, further comprising a filter covering an opening on the organ-on-chip microfluidic device (155) whereby the filter allows the gas phase of vapor, aerosol, or other airborne material to enter the chip.

10. The synthetic organ (100) of claim 1, wherein the one or more mounting positions (140, 141, 142, 143, 144, 145) include discrete mounting points at one or more circumferential positions around the airway.

11. The synthetic organ (100) of claim 1, wherein the organ-on-chip microfluidic device (155) is a single continuous chip that circles the entire inner perimeter of the airway.

12. The synthetic organ (100) of claim 1, wherein the lung tissue or cells (160) is nourished by an oxygen/nutrient solution from below.

13. A method of evaluating a vapor, aerosol, or other airborne material product, the method comprising:
providing the synthetic organ (100) of any one of claims 1 to 12; and
introducing a vapor, aerosol, or other airborne material through a first opening of the synthetic organ (100).

14. The method of claim 13, further comprising evaluating a response of the one or more organ-on-chip microfluidic devices (155) to the vapor, aerosol, or other airborne material.

15. A method of mimicking *in vivo* testing systems for vapor or aerosol products, the method comprising:
providing the synthetic organ (100) of any one of claims 1 to 12; and
introducing aerosol or vapor into the synthetic organ (100).

16. The method of claim 15 further comprising evaluating a response of the one or more organ-on-chip microfluidic devices (155) to the aerosol or vapor.

## Patentansprüche

1. Ein synthetisches Organ (100), umfassend:
einen oder mehrere Luftwege; und
eine erste Öffnung in Kommunikation mit dem einen oder den mehreren Luftwegen, welche dazu ausgebildet ist, einen Dampf, ein Aerosol oder ein anderes von einem luftgetragenen Material gebildetes Aerosol in den einen oder die mehreren Luftwege einzuführen;
eine Organ-on-Chip-Mikrofluidik-Vorrichtung (155), welche Lungengewebe oder -zellen (160) aufweist; und
eine oder mehrere Montageposition(en) (140, 141, 142, 143, 144, 145) innerhalb des einen oder der mehreren Luftwege, welche dazu ausgebildet sind, die Organ-on-Chip-Mikrofluidik-Vorrichtung (155) aufnehmen.

2. Das synthetische Organ (100) nach Anspruch 1 mit mindestens einem von folgenden Merkmalen:
- der eine oder die mehreren Luftwege umfassen einen Tubus;
- der eine oder die mehreren Luftwege umfassen eine oder mehrere Bifurkationen (130);
- der eine oder die mehreren Luftwege bilden mindestens einen Bereich eines Lungendurchlasses nach, wobei insbesondere der Bereich eines Lungendurchlasses repräsentativ für ein Säugetier ist;
- die Wände des einen oder der mehreren Luftwege sind elastisch, wodurch eine Zunahme oder Abnahme eines Drucks innerhalb des einen oder der mehreren Luftwege eine Ausdehnung oder Kontraktion des einen oder der mehreren Luftwege bewirkt;
- der eine oder die mehreren Luftwege umfassen einen Abguss eines Bereichs eines Atemtrakts einer Lunge;
- der eine oder die mehreren Luftwege umfassen einen 3D-Abdruck eines Bereichs eines Atemtrakts einer Lunge;
- der eine oder die mehreren Luftwege sind mit einem elastischen Material aufgebaut.

3. Das synthetische Organ (100) nach Anspruch 1, umfassend eine Mehrzahl von Organ-on-Chip-Mikrofluidik-Vorrichtungen (155), welche über den einen oder die mehreren Luftwege hinweg verteilt sind, wobei jede Organ-on-Chip-Mikrofluidik-Vorrichtung (155) aus der Mehrzahl eine (oder mehrere) Lage(n) von Lungengewebe oder -zellen (160) umfasst, welche nativ ist für den spezifischen Bereich einer Lunge, an dem sie sich befindet.

4. Das synthetische Organ (100) nach Anspruch 3, wobei die Organ-on-Chip-Mikrofluidik-Vorrichtung (155) eine passive Vorrichtung oder eine aktive Vorrichtung ist.

5. Das synthetische Organ (100) nach Anspruch 3, wobei der Dampf, das Aerosol oder das andere luftgetragene Material einen Dampf oder ein Aerosol umfasst, welches Nicotin umfasst (z.B. ein Tabakaerosol).

6. Das synthetische Organ (100) nach Anspruch 3, wobei die Organ-on-Chip-Mikrofluidik-Vorrichtung (155) eine oder mehrere Lagen differenzierten Lungengewebes umfasst.

7. Das synthetische Organ (100) nach Anspruch 1, ferner umfassend eine Kontrollvorrichtung, welche dazu ausgebildet ist, Temperatur, Feuchtigkeit und/oder Konzentration des Dampfes, des Aerosols oder des anderen luftgetragenen Materials innerhalb der Vorrichtung zu kontrollieren.

8. Das synthetische Organ (100) nach Anspruch 1, ferner umfassend eine programmierbare aerosol- oder dampferzeugende Maschine, welche mit der ersten Öffnung zum Zweck der Simulation eines menschlichen Zugprofils in Kommunikation steht.

9. Das synthetische Organ (100) nach Anspruch 3, ferner umfassend ein Filter, welches eine Öffnung an der Organ-on-Chip-Mikrofluidik-Vorrichtung (155) abdeckt, wobei das Filter der Gasphase von Dampf, Aerosol oder einem anderen luftgetragenen Material den Zutritt zu dem Chip gestattet.

10. Das synthetische Organ (100) nach Anspruch 1, wobei die eine oder die mehreren Montagepositionen (140, 141, 142, 143, 144, 145) diskrete Montagepunkte an einer oder mehreren Umfangspositionen um den Luftweg herum umfassen.

11. Das synthetische Organ (100) nach Anspruch 1, wobei die Organ-on-Chip-Mikrofluidik-Vorrichtung (155) ein einziger kontinuierlicher Chip ist, welcher den gesamten inneren Perimeter des Luftwegs umschließt.

12. Das synthetische Organ (100) nach Anspruch 1, wobei das Lungengewebe oder die Lungenzellen (160) mittels einer Sauerstoff-/Nährlösung von unten ernährt werden.

13. Ein Verfahren zum Auswerten eines Dampfes, eines Aerosols oder eines anderen von einem luftgetragenen Material gebildeten Produkts, das Verfahren umfassend:
Bereitstellen des synthetischen Organs (100) nach einem der Ansprüche 1 bis 12; und
Einführen eines Dampfes, eines Aerosols oder eines anderen luftgetragenen Materials durch eine erste Öffnung des synthetischen Organs (100) hindurch.

14. Das Verfahren nach Anspruch 13, ferner umfassend: Auswerten einer Antwort der einen oder der mehreren Organ-on-Chip-Mikrofluidik-Vorrichtungen (155) auf den Dampf, das Aerosol oder das andere luftgetragene Material.

15. Ein Verfahren zum Nachahmen von *in* vivo-Testsystemen für Dampf- oder Aerosolprodukte, das Verfahren umfassend:
Bereitstellen des synthetischen Organs (100) nach einem der Ansprüche 1 bis 12; und
Einführen eines Aerosols oder Dampfes in das synthetische Organ (100).

16. Das Verfahren nach Anspruch 15, ferner umfassend: Auswerten einer Antwort der einen oder der mehreren Organ-on-Chip-Mikrofluidik-Vorrichtungen (155) auf das Aerosol oder den Dampf.

## Revendications

1. Organe synthétique (100) comprenant :
une ou plusieurs voies aériennes ; et
une première ouverture en communication avec la ou les voies aériennes, configurée pour introduire une vapeur, un aérosol, ou un autre matériau en suspension dans l'air dans la ou les voies aériennes;
un dispositif microfluidique de type organe-sur-puce (155) ayant du tissu ou des cellules pulmonaires (160) ; et
une ou plusieurs position(s) de montage (140, 141, 142, 143, 144, 145) à l'intérieur de la ou des voies aériennes configurée(s) pour accepter le dispositif microfluidique de type organe-sur-puce (155).

2. Organe synthétique (100) selon la revendication 1, avec au moins l'un des suivants :
- la ou les voies aériennes comprennent un tube ;
- la ou les voies aériennes comprennent une ou plusieurs bifurcations (130) ;
- la ou les voies aériennes répliquent au moins une partie d'une voie pulmonaire, en particulier dans lequel la partie d'une voie pulmonaire est représentative d'un mammifère ;
- les parois de la ou des voies aériennes sont élastiques, moyennant quoi une augmentation ou une diminution de la pression dans la ou les voies aériennes provoque la dilatation ou la contraction de la ou des voies aériennes ;
- la ou les voies aériennes comprennent une pièce coulée d'une partie d'une voie respiratoire d'un poumon ;
- la ou les voies aériennes comprennent une impression 3D d'une partie d'une voie respiratoire d'un poumon ;
- la ou les voies aériennes est construite en un matériau élastique.

3. Organe synthétique (100) selon la revendication 1, comprenant une pluralité de dispositifs microfluidiques de type organe-sur-puce (155), distribués le long de la ou des voies aériennes, dans lequel chaque dispositif microfluidique de type organe-sur-puce (155) parmi la pluralité comprend une couche(s) de tissu ou cellules pulmonaires (160) natifs de la partie spécifique d'un poumon au niveau de laquelle elle est située.

4. Organe synthétique (100) selon la revendication 3, dans lequel le dispositif microfluidique de type organe-sur-puce (155) est un dispositif passif ou un dispositif actif.

5. Organe synthétique (100) selon la revendication 3, dans lequel la vapeur, l'aérosol, ou un autre matériau en suspension dans l'air, comprend une vapeur ou un aérosol comprenant de la nicotine (par exemple un aérosol de tabac).

6. Organe synthétique (100) selon la revendication 3, dans lequel le dispositif microfluidique de type organe-sur-puce (155) comprend une ou plusieurs couches de tissu pulmonaire différencié.

7. Organe synthétique (100) selon la revendication 1, comprenant en outre un dispositif de commande configuré pour réguler la température, l'humidité et/ou la concentration de la vapeur, de l'aérosol, ou d'un autre matériau en suspension dans l'air, à l'intérieur du dispositif.

8. Organe synthétique (100) selon la revendication 1, comprenant en outre une machine programmable de production d'aérosol ou de vapeur en communication avec la première ouverture destinée à simuler un profil de bouffées humaines.

9. Organe synthétique (100) selon la revendication 3, comprenant en outre un filtre recouvrant une ouverture sur le dispositif microfluidique de type organe-sur-puce (155), moyennant quoi le filtre permet à la phase gazeuse de vapeur, d'aérosol, ou d'autre matériau en suspension dans l'air, d'entrer dans la puce.

10. Organe synthétique (100) selon la revendication 1, dans lequel le ou les positions de montage (140, 141, 142, 143, 144, 145) comprennent des points de montage discrets au niveau d'une ou plusieurs positions circonférentielles autour de la voie aérienne.

11. Organe synthétique (100) selon la revendication 1, dans lequel le dispositif microfluidique de type organe-sur-puce (155) est une seule puce continue qui encercle tout le périmètre intérieur de la voie aérienne.

12. Organe synthétique (100) selon la revendication 1, dans lequel le tissu ou les cellules pulmonaires (160) sont nourris par une solution nutritive/oxygénée depuis le dessous.

13. Procédé d'évaluation d'une vapeur, d'un aérosol, ou d'un autre matériau en suspension dans l'air, le procédé comprenant :
l'obtention de l'organe synthétique (100) de l'une quelconque des revendications 1 à 12 ; et
l'introduction d'une vapeur, d'un aérosol, ou d'un autre matériau en suspension dans l'air à travers une première ouverture de l'organe synthétique (100).

14. Procédé selon la revendication 13, comprenant en outre l'évaluation d'une réponse du ou des dispositifs microfluidiques de type organe-sur-puce (155) à la vapeur, l'aérosol, ou un autre matériau en suspension dans l'air.

15. Procédé d'imitation de systèmes de test *in vivo* pour des produits en vapeur ou en aérosol, le procédé comprenant :
l'obtention de l'organe synthétique (100) de l'une quelconque des revendications 1 à 12 ; et
l'introduction de l'aérosol ou de la vapeur dans l'organe synthétique (100).

16. Procédé selon la revendication 15, comprenant en outre l'évaluation d'une réponse du ou des dispositifs microfluidiques de type organe-sur-puce (155) à l'aérosol ou à la vapeur.
